Europäisches Patentamt

European Patent Office

Office européen des brevets

(11) Veröffentlichungsnummer: **0 517 190 A2**

## EUROPÄISCHE PATENTANMELDUNG

(21) Anmeldenummer: **92109391.0**

(22) Anmeldetag: **03.06.92**

(51) Int. Cl.5: **A61M 13/00**, G05D 16/06

(30) Priorität: **03.06.91 DE 9106790 U**

(43) Veröffentlichungstag der Anmeldung:
**09.12.92 Patentblatt 92/50**

(84) Benannte Vertragsstaaten:
**DE ES FR GB IT NL**

(71) Anmelder: **HIPPOKRATEC Gesellschaft für Medizintechnik mbH**
**Reutweg 5**
**W-8151 Warngau(DE)**

(72) Erfinder: **Fuchs, Manfred**
**Hartpenninger Strasse 2**
**W-8151 Warngau(DE)**
Erfinder: **Meyer, Edgar**
**Griesser 15**
**W-8160 Miesbach(DE)**

(74) Vertreter: **Strehl, Schübel-Hopf, Groening**
**Maximilianstrasse 54 Postfach 22 14 55**
**W-8000 München 22(DE)**

(54) **Insufflationsgerät.**

(57) Bei dem vorliegenden Insufflationsgerät wird der in der Ausgangsleitung 22 des Gerätes herrschende Fluiddruck kontinuierlich abgegriffen und auf den Steuereingang 26 eines in die Fluidleitung eingeschalteten, kontinuierlich steuerbaren Druckreglers 19 rückgekoppelt. Dadurch werden im Normalbetrieb einerseits sowohl jedwede Zeitintervalle, in denen das Fluid unkontrolliert in den Körper insuffliert wird, und andererseits abrupte Druckänderungen vermieden, wie sie durch Ein- und Ausschalten eines Unterbrechers oder durch Umschalten zwischen Strömungswegen mit unterschiedlichen Strömungswiderständen verursacht werden. Ferner ist sichergestellt, daß der Druck in der Ausgangsleitung 22 den am Druckregler 19 eingestellten Vorgabewert nicht übersteigen kann.

*Fig. 1*

Bei endoskopischen Eingriffen in den menschlichen oder tierischen Körper, insbesondere im Unterleibsbereich, ist es erforderlich, den dabei gebildeten Hohlraum mittels eines Fluids, üblicherweise $CO_2$-Gas, aufzudehnen. Dadurch entsteht ein Freiraum, der es dem Operateur erlaubt, die Eingriffe über geeignete Zugänge in der Körperhöhle auszuführen.

Aus DE-U-7 611 305 ist ein Gasinsufflationsgerät bekannt, das mit einer bifilaren, koaxialen Veress-Nadel arbeitet. Während das Gas unter Druck durch die innere Kanüle in die Körperhöhle eingeleitet wird, ist die koaxiale äußere Kanüle an einen Druckschalter angeschlossen, der ein in der Gaszuführung vorgesehenes Ventil ausschaltet, wenn der Druck in der Körperhöhle einen vorgegebenen Wert erreicht.

Das Hauptproblem dieses Geräts besteht in der Gefahr einer Verstopfung der Meßöffnung der Koaxialnadel, was dazu führt, daß der Druckschalter nicht anspricht und die fortgesetzte Gaszuführung in der Körperhöhle einen unzulässig hohen Druck erzeugt.

Darüberhinaus entstehen auch bei ordnungsgemäßer Arbeitsweise durch das Ein- und Ausschalten des Ventils abrupte Druckschwankungen, die zu entsprechenden Bewegungen der Hohlraumwandung führen und die Arbeit des Operateurs stören. Wird die Hysterese der Regelschleife zur Vermeidung unkontrollierter Oszillationen genügend groß gemacht, so sind diese Druckschwankungen entsprechend ausgeprägt.

Bei einem weiteren, aus DE-A-3 413 631 bekannten Insufflationsgerät, das die im ersten Teil des Anspruchs 1 angegebenen Merkmale aufweist, wird mit einer einläufigen Nadel gearbeitet, so daß die Gefahr eines unzulässigen Druckanstiegs in der Körperhöhle infolge Verstopfens einer separaten Meßöffnung beseitigt ist. In diesem Fall wird der Druck an dem mit der monofilaren Insufflationsnadel verbundenen Geräteausgang gemessen, wobei das elektrische Ausgangssignal des Druckmessers in einem Rechner weiterverarbeitet und dann, wenn das weiterverarbeitete Signal einen vorgegebenen Grenzwert überschreitet, ein Ausgangssignal erzeugt wird, das einen in der Gaszuführleitung vorgesehenen Unterbrecher betätigt.

Auch in diesem Fall ergeben sich die oben geschilderten, aus dem abrupten Unterbrechen und Wiedereinschalten der Gasströmung resultierenden Probleme.

Ein weiterer Nachteil besteht darin, daß der am Geräteausgang abgegriffene Druck nicht unmittelbar zur Betätigung des Unterbrechers herangezogen wird, sondern in ein elektrisches Signal umgewandelt wird, das um einen beim Einschalten des Gerätes ermittelten, dem Widerstand zwischen dem Geräteausgang und der Körperhöhle entsprechendem Grundwert verändert wird. Ändert sich im Laufe der Operation der tatsächlich vorhandene Widerstand, so wird der Unterbrecher bei einem Druck betätigt, der nicht mehr dem vorgesehenen Druck entspricht.

Eine weitere Schwierigkeit besteht darin, daß die Genauigkeit der Abschaltung nicht nur durch das Verhalten des Unterbrechers selbst, sondern auch durch zeitliche Änderungen oder Störungen des Druckmessers und des Rechners beeinflußt wird.

Aus DE-A-3 739 003 ist ein weiteres Insufflationsgerät bekannt, bei dem die Gaszufuhr abwechselnd über zwei verschiedene Strömungswege erfolgt. Der eine Strömungsweg weist einen verhältnismäßig niedrigen Strömungswiderstand auf und dient zum schnellen Füllen der Körperhöhle. Im anderen Strömungsweg sind ein einstellbarer Druckminderer zur Vorgabe eines höheren Strömungswiderstandes sowie ein Strömungsmeßgerät vorgesehen. Immer dann, wenn bei Einschalten des zweiten Strömungsweges das Strömungsmeßgerät eine Strömungsgeschwindigkeit feststellt, die über einem vorgegebenen Grenzwert liegt (was bedeutet, daß der Druck in der Körperhöhle einen vorgegebenen Druck noch nicht erreicht hat), wird ein Umschaltventil angesteuert, das jeweils für eine fest vorgegebene Zeitspanne den ersten Strömungsweg einschaltet und während dieser Zeitspanne zu einem schnelleren Füllen der Körperhöhle führt.

Auch bei dieser Steuerung ergeben sich aufgrund des abrupten Umschaltens zwischen zwei Strömungswegen mit stark unterschiedlichen Strömungswiderständen entsprechend abrupte Druckwellen mit den oben bereits erläuterten Nachteilen.

Ein weiteres Problem besteht darin, daß während des durch das zeitgesteuerte Umschaltventil fest vorgegebenen Zeitintervalls, innerhalb der die Schnellfüllung erfolgt, keine Druckmessung erfolgt, so daß eine Überfüllung der Körperhöhle auf einen unzulässig hohen Druck nicht mit Sicherheit vermieden wird.

Das gleiche Problem besteht bei einem weiteren, aus DE-C-3 611 018 bekannten Gerät. Hier wird im Normalbetrieb ein steuerbarer Druckminderer zwischen einem Solldruck und einem Maximaldruck hin- und hergesteuert, wobei die Führungsgröße des Druckmiderers die von einem Strömungsmeßgerät erfaßte Strömungsgeschwindigkeit ist. Immer dann, wenn der Druckminderer auf den Solldruck eingestellt ist und die Strömunsgeschwindigkeit einen vorgegebenen Wert überschreitet, führt eine Steuerschaltung während einer von einem Taktgeber vorgegebenen Zeitspanne den Druckminderer in seine Maximaldruckstellung, so daß während dieser Zeitspanne eine Schnellfüllung der Körperhöhle erfolgt. Während des Betriebs wird von Zeit zu Zeit ein Druckmeßzyklus eingeschaltet, bei dem der Druckminderer so weit heruntergefahren wird, daß sein Ausgangsdruck dem Druck in der Körperhöhle entspricht; in diesem dann hergestellten strömungslosen Zustand erfolgt eine statische Druckmessung.

Wiederum sind im Normalbetrieb wegen des abrupten Umschaltens des Druckminderers zwischen Solldruck und Maximaldruck die erläuterten Druckschwankungen zu beobachten, und wiederum ist nicht ausgeschlossen, daß innerhalb des von dem Taktgeber fest vorgegebenen Zeitintervalls, in dem eine Schnellfüllung erfolgt, ein unzulässig hoher Druck erreicht wird.

Der Erfindung liegt die Aufgabe zugrunde, ein Insufflationsgerät anzugeben, bei dem der Druck am Geräteausgang ohne zwischengeschaltete Steuerelektronik und unter Vermeidung von abrupten Druckschwankungen automatisch und kontinuierlich auf einem fest vorgebbaren Wert gehalten wird, der dem in dem Körperhohlraum gewünschten bzw. zulässigen Druck entspricht.

Die erfindungsgemäße Lösung dieser Aufgabe ist im Anspruch 1 gekennzeichnet. Danach wird der in die Fluidleitung eingefügte Druckregler an seinem Steuereingang mit dem Fluiddruck am Geräteausgang beaufschlagt. Der Druck am Geräteausgang wird also ständig abgegriffen und zur kontinuierlichen Steuerung des Druckreglers herangezogen. Bei dem erfindungsgemäßen Gerät erfolgt im Normalbetrieb keine abrupte Unterbrechung oder Umschaltung zwischen unterschiedlichen Druckwerten oder Strömungswegen mit unterschiedlichen Widerständen. Daher treten auch keine Druckschwankungen auf.

Der Einsatz eines Druckreglers mit externer Signalrückkopplung von der Ausgangsleitung ermöglicht ein Überwinden der dazwischen liegenden Strömungswiderstände durch den Fluiddruck. Daher liegt am am Ausgang des Geräts ein Druck, der nicht höher sein kann als der am Druckregler eingestellte Druck, wobei dieser höchstens den Wert annehmen kann, der von einem davor liegenden Druckminderer zur Verfügung gestellt wird.

Das erfindungsgemäß angewandte Niederdruckprinzip bietet zusätzlich die Sicherheit, daß beim ersten Einstechen der Insufflationsnadel etwa durch die Bauchdecke hindurch ein versehentliches Einleiten des Fluids ($CO_2$) in Venen oder Gewebeteile nicht möglich ist. Der dafür erforderlichen Druck liegt nämlich über dem Insufflationsdruck und wird bei dem erfindungsgemäßen Gerät nicht erreicht.

Bei der nach Anspruch 2 vorgesehenen unmittelbaren Rückkopplung des Fluiddrucks vom Geräteausgang auf den Steuereingang des Druckreglers sind Fehlerquellen und Störeinflüsse, wie sie aus der Umwandlung von Druck in elektrische Signale oder bei der Signalverarbeitung in einer Steuerelektronik auftreten können, vermieden.

In der Weiterbildung der Erfindung nach Anspruch 3 lassen sich die Bezugsgröße für den Druckregler und damit der am Geräteausgang maximal erreichbare Druck vorgeben, so daß das Maß der Aufdehnung der Körperhöhle je nach den Erfordernissen eingestellt werden kann.

Eine bevorzugte Gestaltung des Druckreglers ist in den Ansprüchen 4 und 13 bis 15 angegeben. Danach ist der Steuerteil von dem in die Fluidleitung eingeschalteten gesteuerten Teil des Druckreglers strömungsmäßig getrennt. Ferner läßt sich der Sollwert des Druckreglers über die Verstellung einer Federkraft stufenlos, gegebenenfalls über eine Steuerschaltung, verändern.

Mittels der nach den Ansprüchen 5 und 6 vorgesehenen verstellbaren Strömungsdrossel lassen sich die im Gerät auftretenden Differenzdrücke an die Arbeitsbereiche handelsüblicher Drucksensoren anpassen. Im übrigen ergibt sich bei dem erfindungsgemäßen Geräät eine Strömungsbegrenzung automatisch durch den jeweiligen Füllzustand der Körperhöhle.

Besonders einfache und gleichzeitig präzise Möglichkeiten der Veränderung des Strömungsquerschnittes sind in den Ansprüchen 7 und 8 angegeben. Dabei wird eine Verstellcharakteristik erreicht, bei der bei konstanter Beaufschlagung des Stellgliedes bei kleinem Öffnungsquerschnitt eine geringere Änderungsgeschwindigkeit als bei großem Öffnungsquerschnitt erreicht wird. Die Gestaltung nach Anspruch 9 zeigt eine baulich einfach Möglichkeit der Wahl zwischen zwei fest vorgegebenen Strömungsquerschnitten.

Gemäß Anspruch 10 läßt sich der Strömungsbegrenzer ferner in Verbindung mit einem Differenzdruckmesser zur Ermittlung der Durchflußmenge heranziehen.

Anspruch 11 betrifft eine zusätzliche Sicherheitsmaßnahme für den Fall, daß der Druckregler selbst fehlerhaft arbeitet, um ein Überschreiten eines vorgegebenen zulässigen Druckes mit Sicherheit zu unterbinden.

Die Ausgestaltung nach Anspruch 12 gestattet es ferner, das erfindungsgemäße Gerät auch zur Strömungsregelung heranzuziehen.

Ausführungsbeispiele der Erfindung werden nachstehend anhand der Zeichnungen näher erläutert. Darin zeigt
Figur 1
ein Schaltschema des gesamten Geräts,
Figur 2
eine schematische Darstellung eines bevorzugten Druckreglers,
Figur 3A, 3B; 4A, 4B und 5A, 5B
schematische Darstellungen von Strömungsdrosseln, jeweils gesehen von der Seite und in Strömungsrichtung.

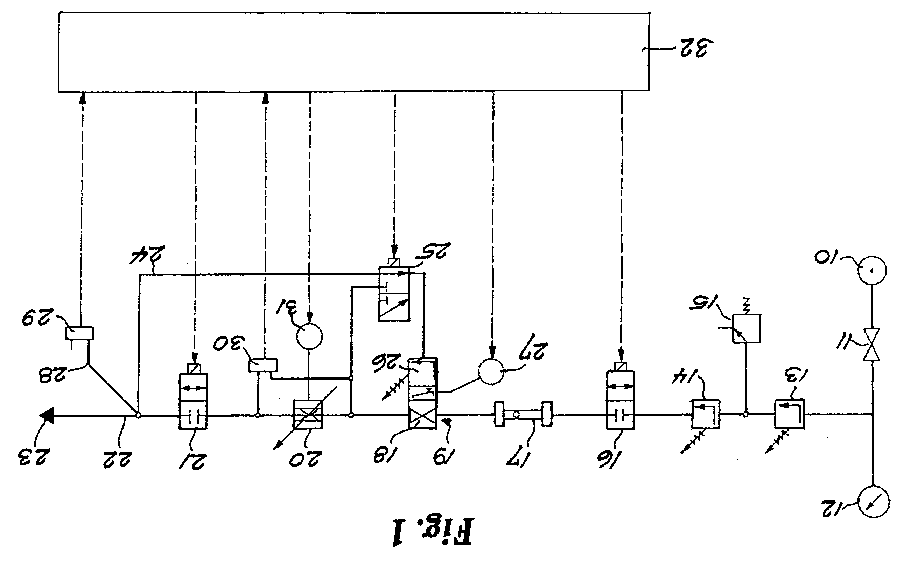

Gemäß Figur 1 umfaßt die Vorrichtung einen Druckgasbehälter 10 mit einem Absperrventil 11 und einem Manometer 12 zur Anzeige des Füllzustandes des Druckgasbehälters 10. Nach Öffnen des Absperrventils 11 gelangt das unter hohem Druck stehende Gas zu einem Druckminderer 13, der das Gas auf einen mittleren Druck von beispielsweise 6 bar entspannt. In einem zweiten Druckminderer 14 wird das noch unter Druckschwankungen stehende Gas auf einen präzisen Druck von beispielsweise 250 mbar weiter entspannt. Zur Vermeidung von unzulässig hohen Ausgangsdrücken des ersten Druckminderers 13 ist an dessen Ausgang ferner ein Überdruckventil 15 angeschlossen.

Das auf etwa 250 mbar entspannte Gas durchströmt nun nacheinander ein Magnetventil 16, einen Durchflußanzeiger 17, die Hochdruckkammer 18 eines Druckreglers 19, eine Strömungsdrossel 20 und ein weiteres Magnetventil 21 und gelangt über eine Ausgangsleitung 22 zum Geräteausgang 23, an den über eine (nicht gezeigte) Schlauchleitung das eigentliche (ebenfalls nicht gezeigte) Insufflationsinstrument angeschlossen ist, bei dem es sich um eine Veress-Nadel oder ein Trokar handeln kann und über das das Gas in die Körperhöhle eingeleitet wird.

Von der Ausgangsleitung 22 zweigt eine Fluidleitung 24 ab, die über ein magnetisch betätigbares Umschaltventil 25 an die Steuerkammer 26 des Druckreglers 19 angeschlossen ist. Die Bezugsgröße für den Druckregler 19 wird über einen Stellmotor 27 eingegeben. In seiner anderen Stellung verbindet das Umschaltventil 25 die Steuerkammer 26 des Druckreglers 19 mit der Ausgangsseite der Hochdruckkammer 18 des Druckreglers 19.

Von der Ausgangsleitung 22 zweigt eine weitere Fluidleitung 28 ab, die mit einem Druckmesser 29 verbunden ist.

Die beiden Eingänge eines Differenzdruckmessers 30 sind mit dem Eingang bzw. dem Ausgang der Strömungsdrossel 20 verbunden. Die Strömungsdrossel 20 ist über einen Stellmotor 31 verstellbar.

Wie ersichtlich weist das Gerät nur einen einzigen Strömungspfad für das zu insufflierende Gas auf, während alle anderen (teilweise noch zu erläuternden) abzweigenden Fluidleitungen lediglich zu Regel- und Meßzwecken dienen.

In Figur 1 ist ferner schematisch eine gemeinsame Steuerschaltung 32 vorgesehen, an der die elektrischen Ausgangssignale des Druckmessers 29 und des Differenzdruckmessers 30 liegen, und die die elektrischen Steuersignale für die Ventile 16, 21 und 25 sowie für die Stellmotoren 27 und 31 liefert. Die Steuerschaltung enthält die für einen automatischen Programmablauf erforderliche Elektronik sowie Bedienungselemente zur gegebenenfalls manuellen Vorgabe von Druck- und Strömungswerten und Anzeigeinstrumente für die gemessenen Drücke.

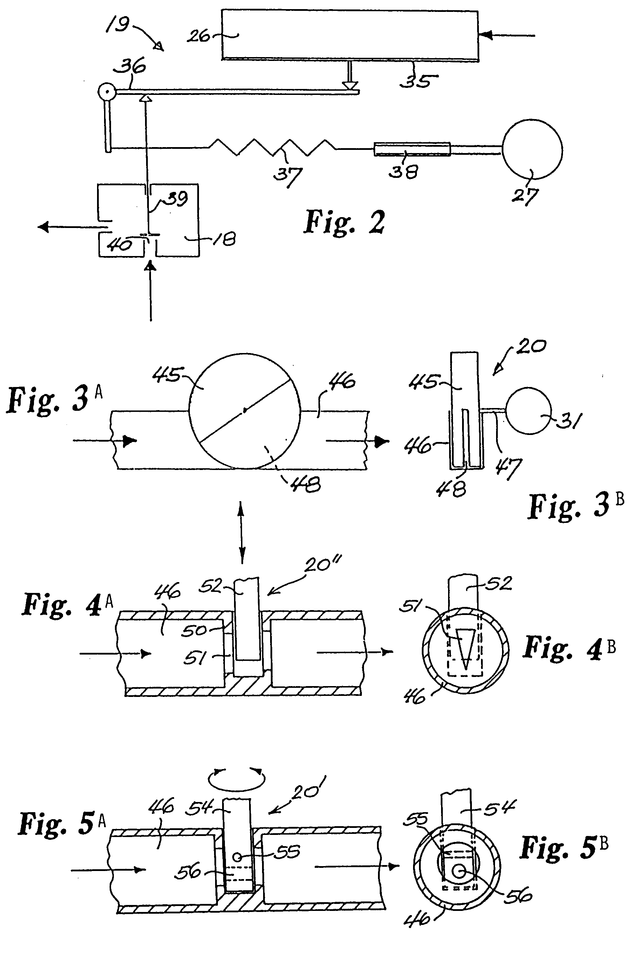

Gemäß der schematischen Darstellung des Druckreglers 18 nach Figur 2 ist die über das Umschaltventil 25 mit der Ausgangsleitung 22 (bzw. dem Ausgang des Druckreglers selbst) in Verbindung stehende Steuerkammer 26 mit einer Membran 35 verschlossen, deren Auslenkung auf den langen Arm eines zweiarmigen Hebels 36 wirkt. Am anderen wesentlich kürzeren Arm dieses Hebels 36 greift eine Rückstellfeder 37 an, deren Vorspannung über ein Spanngewinde 38 von dem Stellmotor 27 einstellbar ist. Je stärker die Rückstellfeder 37 gespannt ist, desto größer muß der auf die Membran 35 wirkende Gasdruck sein, um den an einem kurzen Arm des Hebels 36 angreifenden Ventilkörper 39 zu verstellen, der zum graduellen Öffnen bzw. Schließen einer in der Hochdruckkammer 18 vorgesehenen Strömungsöffnung 40 dient.

Die in Figur 3A und 3B schematisch dargestellte Strömungsdrossel 20 weist eine kreiszylindrische Scheibe 45 auf, die zur Hälfte in ein im Zuge der Fluidleitung vorgesehenes Rechteckrohr 46 eintaucht und um eine zur Strömungsrichtung senkrechte Achse 47 verschwenkbar ist. Die Scheibe 45 weist einen zu ihren Stirnflächen parallelen mittleren Segmentschlitz 48 auf, der durch die Hälfte der Scheibe verläuft.

Über den Stellmotor 31 läßt sich die Scheibe 45 zwischen einer ersten Stellung, in der die innere gerade Begrenzung des Schlitzes 48 in Strömungsrichtung verläuft und die Drossel maximale Öffnung aufweist, und einer gegenüber dieser ersten Stellung um mindestens 90° gedrehten zweiten Stellung verschwenken, in der der nichtgeschlitzte Bereich der Scheibe 45 den Strömungsweg völlig verschließt.

Bei der in Figur 4A und 4B dargestellten Ausführungsform der Strömungsdrossel 20' ist in der in diesem Fall kreiszylindrischen Fluidleitung 46 senkrecht zur Strömungsrichtung eine Verengung in Form einer Blendenplatte 50 mit einem V-Schlitz 51 vorgesehn. Stromabwärts von der Blendenplatte 50 ist ein Stempel 52 angeordnet, der mittels eines Stellmotors und eines Exzenters (nicht gezeigt) parallel zu der Blendenplatte 50 derart verschiebbar ist, daß er den Schlitz 51 mehr oder weniger freigibt. Der Scheitelwinkel und die Gestalt des Schlitzes 51 bestimmen die Charakteristik der Querschnittsveränderung. Seine V-Form ergibt gegenüber linearen Stellgliedern eine präzise Steuerung insbesondere auch im Bereich geringer Querschitte.

Eine weitere Variante zur Veränderung des Strömungsquerschnitts stellt die Strömungsdrossel 20'' nach Figur 5A und 5B dar. Hier ist in der Fluidleitung 46 ein senkrecht zur Strömungsrichtung stehender Zapfen 54 vorhanden, der zwei um 90° versetzte Bohrungen 55, 56 unterschiedlicher Durchmesser aufweist und mittels eines (wiederum nicht gezeigten) Motors um seine Achse drehbar gelagert ist. In diesem Fall läßt sich der Strömungsquerschnitt auf jeweils einen von zwei fest vorgegebenen Werten einstellen.

In der in Figur 1 gezeigten Stellung des Umschaltventils 25 wirkt der in der Ausgangsleitung 22 herrschende Gasdruck auf die Membran 35 der Steuerkammer 26 des Druckreglers 19. Dadurch wird der mit der Öffnung 40 in der Hochdruckkammer 18 zusammenwirkende Ventilkörper 39 in eine Stellung gebracht, die von der über den Stellmotor 27 vorgewählten Spannung der Rückstellfeder 37 abhängt.

Erreicht der Druck in der Ausgangsleitung 22 den vorgewählten Grenzdruck, mit dem der Hohlraum im Körper des Patienten aufgeweitet werden soll, so wird die Öffnung 40 geschlossen. Wegen der während einer Operation ständig auftretenden Leckverluste wird dieser Druck normalerweise jedoch nicht vollständig erreicht, so daß auch die Öffnung 40 in der Hochdruckkammer 18 nicht vollständig geschlossen wird.

Sinkt der Druck in Ausgangsleitung 22 etwa infolge größerer Leckverluste stärker ab, so wird die Öffnung 40 in der Hochdruckkammer 18 entsprechend weiter geöffnet, was wiederum zu einer Druckerhöhung führt. Die beschriebene Regelschleife bewirkt also eine automatische Einregelung des in der Ausgangsleitung 22 herrschenden Drucks auf den über den Stellmotor 27 am Druckregler 19 eingestellten Wert.

Bei Versagen der Regelung etwa infolge einer Störung des Druckreglers 19 selbst wird der dann in der Ausgangsleitung 22 weiter ansteigende Druck vom Druckmesser 29 erfaßt, der in der Steuerschaltung 32 ein Signal zur Betätigung des Magnetventils 21 auslöst. Auf diese Weise werden eine weitere Gaszufuhr und damit eine weitere Druckerhöhung mit Sicherheit vermieden.

Da das Magnetventil 21 in der Fluidleitung in Serie liegt, wird gleichzeitig die in die Körperhöhle führende Leitung verschlossen, so daß auch eine plötzliche Entleerung der Körperhöhle vermieden wird. Eine derartige plötzliche Entleerung könnte zu Verletzungen durch die in die Körperhöhle eingebrachten Instrumente führen.

Die Strömungsdrossel 20 läßt sich von der Steuerschaltung 32 über den Stellmotor 31 auf einen definierten Strömungswiderstand einstellen, wie er zur exakten Messung des Differenzdruckes mittels des Druckmessers 30 erforderlich ist. Über die Steuerschaltung 32 kann das Ausgangssignal des Differenzdruckmessers 30 zur Beaufschlagung des Stellmotors 31 der Strömungsdrossel 20 herangezogen werden.

In der in Figur 1 gezeigten Ruhestellung des Umschaltventils 25 lassen sich Durchflußraten von etwa 2 bis 12 ℓ/min erreichen. Zur Erzielung geringerer Durchflußraten von etwa 0,1 bis 2 ℓ/min wird das Umschaltventil 25 in seine andere Stellung gesteuert. In dieser Anordnung kann durch die Strömungsdrossel 20 ein zusätzlicher Staudruck erzeugt werden, der den Druckregler 19 wesentlich früher zur Begrenzung der Gasströmung veranlaßt. In dieser zweiten Stellung des Umschaltventils 25 arbeitet die Schaltung in einer Strömungsregel-Charakteristik.

## Patentansprüche

1. Gerät zum Insufflieren eines Fluids in den Körper eines Patienten mit

    einer Fluidquelle (10), die das Fluid mit einem über dem im Körperinnern zu erzielenden Druck zur Verfügung stellt, und

    einer zwischen die Fluidquelle (10) und eine Ausgangsleitung (22) des Gerätes eingeschalteten, in Abhängigkeit vom Druck in der Ausgangsleitung (22) gesteuerten Einrichtung (19) zur Beeinflussung des Fluiddrucks,

    dadurch gekennzeichnet, daß die den Fluiddruck beeinflussende Einrichtung ein kontinuierlich steuerbarer Druckregler (19) ist, dessen Steuereingang (26) an die Ausgangsleitung (22) angeschlossen ist.

2. Gerät nach Anspruch 1, dadurch gekennzeichnet, daß der Steuereingang (26) des Druckreglers (19) unmittelbar an die Ausgangsleitung (22) angeschlossen ist.

3. Gerät nach Anspruch 1 oder 2, dadurch gekennzeichnet, daß der Druckregler (19) so einstellbar ist, daß er die Fluidleitung schließt, wenn der Druck in der Ausgangsleitung (22) einen Vorgabewert erreicht.

4. Gerät nach Anspruch 3, dadurch gekennzeichnet, daß der Druckregler (19) umfaßt:

    eine mit der Ausgangsleitung (22) verbundene, von einer Membran (35) verschlossene Steuerkammer (26),

    einen von der Membran (35) gegen die Kraft einer Rückstellfeder (37) verschwenkbaren Hebel (36),

    ein in der Fluidleitung angeordnetes Ventil (39, 40) mit einem entsprechend der Hebelbewegung kontinuierlich steuerbaren Öffnungsgrad, und

    ein Stellglied (27, 38) zur Änderung der Kraft der Rückstellfeder (37).

**5.** Gerät nach einem der Ansprüche 1 bis 4, dadurch gekennzeichnet, daß zwischen den Druckregler (19) und die Ausgangsleitung (22) eine Strömungsdrossel (20) eingeschaltet ist.

**6.** Gerät nach Anspruch 5, dadurch gekennzeichnet, daß der Strömungsquerschnitt der Strömungsdrossel (20) über einen von einer Steuerschaltung (32) betätigbaren Stellmotor (31) änderbar ist.

**7.** Gerät nach Anspruch 5 oder 6, dadurch gekennzeichnet, daß die Strömungsdrossel (20) eine in die Fluidleitung (46) teilweise eintauchende, um eine zur Strömungsrichtung senkrechte Achse (47) verdrehbare Scheibe (45) aufweist, die in einer zur Achse (47) senkrechten Ebene mit einem Segmentschlitz (48) versehen ist.

**8.** Gerät nach Anspruch 5 oder 6, dadurch gekennzeichnet, daß die Strömungsdrossel (20') einen in der Fluidleitung (46) angeordneten V-Schlitz (51) enthält, der durch einen quer zur Strömungsrichtung verschiebbaren Stempel (52) kontinuierlich verschließbar ist.

**9.** Gerät nach Anspruch 5 oder 6, dadurch gekennzeichnet, daß die Strömungsdrossel (20'') einen in die Fluidleitung (46) eintauchenden drehbaren Zapfen (54) aufweist, der von zwei quer zueinander verlaufende Bohrungen unterschiedlichen Querschnitts durchsetzt ist.

**10.** Gerät nach einem der Ansprüch 6 bis 9, gekennzeichnet durch einen Differenzdruckmesser (30), dessen beide Eingänge mit der stromaufwärtigen und der stromabwärtigen Seite der Strömungsdrossel (20) verbunden sind und dessen Ausgangssignal an der Steuerschaltung (32) liegt.

**11.** Gerät nach einem der Ansprüche 1 bis 10, dadurch gekennzeichnet, daß an die Ausgangsleitung (22) ein Druckmesser (29) angeschlossen ist, dessen Ausgangssignal bei Überschreiten eines Vorgabewertes ein mit der Ausgangsleitung (22) in Serie geschaltetes Unterbrecherventil (21) betätigt.

**12.** Gerät nach einem der Ansprüche 1 bis 11, gekennzeichnet durch ein Umschaltventil (25) zum wahlweisen Anschluß des Steuereingangs (26) des Druckreglers (19) an die Ausgangsleitung (22) oder an den Druckreglerausgang.

**13.** Druckregler zur Steuerung des in einer Fluidleitung herrschenden Druckes, gekennzeichnet durch

eine mit einer Ausgangsleitung (22) verbundene, von einer Membran (35) verschlossene Steuerkammer (26),

einen von der Membran (35) gegen die Kraft einer Rückstellfeder (37) verschwenkbaren Hebel (36),

ein in der Fluidleitung angeordnetes Ventil (39, 40) mit einem entsprechend der Hebelbewegung kontinuierlich steuerbaren Öffnungsgrad, und

ein Stellglied (27, 38) zur Änderung der Kraft der Rückstellfeder (37).

**14.** Gerät nach Anspruch 4 oder 13, dadurch gekennzeichnet, daß die Rückstellfeder (37) an einem wesentlich kürzeren Hebelarm angreift als die Membran (35).

**15.** Gerät nach einem der Ansprüche 4, 13 und 14, dadurch gekennzeichnet, daß das Stellglied einen von einer Steuerschaltung (32) betätigbaren Stellmotor (27) aufweist.

Fig. 1

*Fig. 2*

*Fig. 3* A

*Fig. 3* B

*Fig. 4* A

*Fig. 4* B

*Fig. 5* A

*Fig. 5* B